# EUROPEAN PATENT APPLICATION

(11) **EP 3 616 742 A1**
(43) Date of publication of application: **04.03.2020**
(21) Application number: 18791083.1
(22) Date of filing: 26.04.2018
(51) Int. Cl.: A61M 27/00, A61M 25/09, A61F 2/04, A61F 2/95

(54) **DRAINAGE CATHETER**

(30) Priority: 28.04.2017 KR 20170054822
(71) Applicant: S&G Biotech, Inc., Yongin-si, Gyeonggi-do 17023 (KR)
(72) Inventor: KANG, Sung Kwon, Yongin-si Gyeonggi-do 16876 (KR); PARK, Sun Soon, Seongnam-si Gyeonggi-do 13559 (KR); JUNG, So Hee, Gwangju-si Gyeonggi-do 12766 (KR)
(74) Representative: Kransell & Wennborg KB
(86) International application number: PCT/KR2018/004852
(87) International publication number: WO 2018/199648

(57) **Abstract**

The drainage catheter according to the examples of the present invention comprises a first induction conduit; and a second induction conduit having a circumference part surrounding the first induction conduit, in which a cut part is formed so that a front edge of the first induction conduit is exposed to the outside.

## Description

### [TECHNICAL FIELD]

The present invention relates to a drainage catheter, and more specifically, to a drainage catheter that is installed in a branched bile duct to allow smooth discharge of bile.

### [BACKGROUND ART]

Typically, when stenosis occurs in an organ that needs to have a certain internal diameter such as blood vessels, esophagus or bile ducts due to sediment or the like, a stent or catheter may be used to artificially expand the narrowed region.

In particular, when stenosis occurs in a bile duct, which functions as a path for the movement of bile, a digestive enzyme produced in the liver, the diameter of the bile duct becomes narrower, which may hinder smooth discharge of bile.

Therefore, a catheter may be inserted into the narrowed bile duct to widen the bile duct to have bile smoothly discharged, thereby preventing backflow of food or fluid.

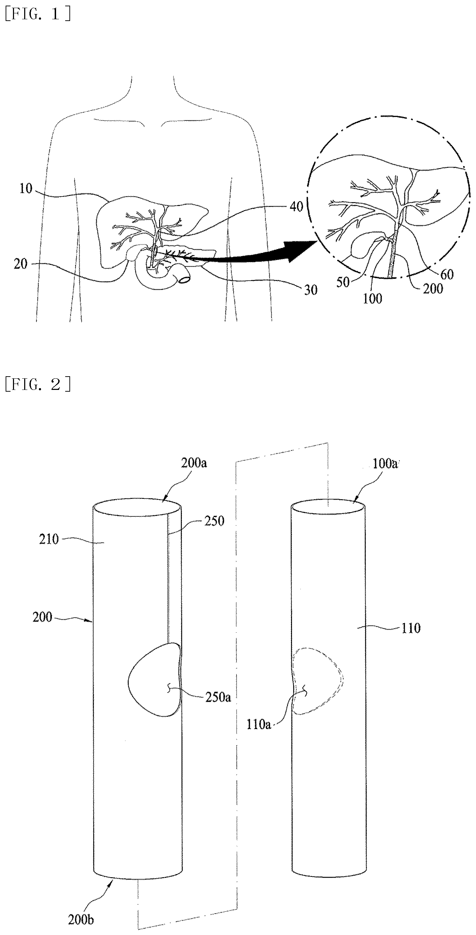

Referring to Fig. 1, bile ducts (40) may connect between the liver (10), the gallbladder (20), and the pancreas (30), and the bile ducts (40) have Y-shaped branching points. When installing a catheter in the bile ducts (40), it may be difficult to install a catheter at such branching points.

A prior art document (Korean Patent Laid-Open No. 10-2012-0035978) describes installation of a stent at a branching point in the bile ducts (40) as described above. In the prior art document, stents were sequentially installed in a first branch duct and a second branch duct using an insertion device.

However, when stents are installed in a first branch duct and second branch, respectively, as in the prior art document, it is difficult, and take long, to install them, and a flow of bile may not be good at the points where two stents are coupled.

### [Prior art document]

### [Patent document]

**(Patent document 0001)** Korean Patent Laid-Open No. 10-2016-0001691 (publication date: January 6, 2016)

### [DETAILED DESCRIPTION OF THE INVENTION]

### [PROBLEM TO BE SOLVED]

The present invention, which is aimed to solve the problems mentioned above, intends to provide drainage catheters that can be inserted as coupled in a bile duct, and when separated, installed in branch bile ducts, respectively.

The object of the present invention is not limited to the above-mentioned object, and other objects that are not mentioned will be clearly understood by those skilled in the art from the following description.

### [MEANS FOR SOLVING THE PROBLEM]

According to an aspect of the present invention, a drainage catheter is provided that comprises a first induction conduit; and a second induction conduit having a circumference part surrounding the first induction conduit, in which a cut part is formed so that a front edge of the first induction conduit is exposed to the outside.

In a drainage catheter according to an aspect of the present invention, the cut part may be formed extending from a front edge toward a rear edge of the second induction conduit in the longitudinal direction, and a fixing hole penetrating the circumference part may be formed on the cut part.

Additionally, the fixing hole may be formed with a size corresponding to that of the first induction conduit, so that the fixing hole surrounds the first induction conduit.

Additionally, the first induction conduit may have a hollow shaped body part in contact with the circumference part of the second induction conduit, and an opening hole may be formed on a face of the body part in opposition to the penetrating hole of the second induction conduit.

Additionally, a protrusion part may be formed in a region of the body part of the first induction conduit, the region corresponding to the cut part of the second induction conduit.

Additionally, the protrusion part may be formed such that the width of an edge contacting the cut part is narrower than that of the other edge connected to the body part.

Additionally, the body part of the first induction conduit may be formed such that a side contacting the cut part is protruded outwardly in comparison to the other side that is opposite in the radial direction.

Additionally, the circumference part of the second induction conduit may be formed such that a face constituting the cut part overlaps onto an upper portion of the other face.

Additionally, the circumference part of the second induction conduit may be formed such that a side on which the cut part is formed is protruded outwardly in comparison to the other side that is opposite in the radial direction.

Additionally, the first induction conduit may have a hollow shaped body part in contact with the circumference part of the second induction conduit, and the drainage catheter may further comprise: a first guide wire which is inserted into the body part and is exposed from the body part to guide the first induction conduit to an installation location; and a second guide wire which is inserted into a separated space between the circumference part and the body part and is exposed from the circumference part to guide the second induction conduit to an installation location.

### [EFFECT OF THE INVENTION]

The drainage catheters according to the examples of the present invention exhibit the following effects.

First, the first induction conduit and the second induction conduit are coupled and then, inserted together as coupled in a bile duct to allow easier installation and reduced procedure time compared with separate insertion of the first induction conduit and the second induction conduit.

Second, with a cut part formed in the second induction conduit, the first induction conduit is easily separated from the second induction conduit to allow easy installation of the first induction conduit and the second induction conduit at a branching point of a bile duct.

Third, with an opening hole formed in the first induction conduit to communicate with the second induction conduit, both bile introduced through the first induction conduit and bile introduced through the second induction conduit can be smoothly discharged to a bile duct.

The effects of the present invention are not limited to the above-mentioned effects, and other effects that are not mentioned will be clearly understood by those skilled in the art from the description of the claims.

### [BRIEF DESCRIPTION OF THE DRAWINGS]

Fig. 1 is a drawing showing the drainage catheter of the present invention being installed in a bile duct.
Fig. 2 is an exploded perspective view of the drainage catheter according to an example of the present invention.
Figs. 3 and 4 are perspective views showing a first induction conduit of the drainage catheter according to an example of the present invention as coupled to a second induction conduit and as separated from the second induction conduit.
Figs. 5 to 7 are drawings showing modified examples of the first induction conduit of the drainage catheter according to examples of the present invention.
Figs. 8 and 9 are drawings showing modified examples of the second induction conduit of the drainage catheter according to examples of the present invention.
Fig. 10 is a perspective view showing a first guide wire and second guide wire coupled to the drainage catheter according to an example of the present invention.
And, Figs. 11 to 13 are drawings to describe the process of installation of the drainage catheter according to an example of the present invention in a bile duct.

### [DETAILED DESCRITPION TO CARRY OUT THE INVENTION]

Hereinafter, examples of the present invention are described in detail with reference to the accompanying drawings. However, those having ordinary skill in the pertinent technical field will easily understand that the accompanying drawings are only described in order to more easily disclose the content of the present invention and that the scope of the present invention is not limited to the scope of the accompanying drawings.

The drainage catheter according to an example of the present invention, which is installed in a bile duct (40) to broaden the bile duct (40), may be installed at a branched section of the bile duct (40).

Specifically, referring to Figs. 1 to 3, the drainage catheter according to an example of the present invention comprises a first induction conduit (100) and a second induction conduit (200).

The first induction conduit (100) and the second induction conduit (200) can be inserted, as coupled, into the bile duct (40), such that the drainage catheter according to an example of the present invention may be easily moved to an installation location.

When the installation location is reached, the first induction conduit (100) and the second induction conduit (200) may be separated and respectively installed in a first branch bile duct (50) and a second branch bile duct (60) where the bile duct (40) diverges.

Specifically, the second induction conduit (200) may have a circumference part (210) surrounding the first induction conduit (100), where a cut part (250) may be formed so that a front edge (100a) of the first induction conduit (100) is exposed to the outside.

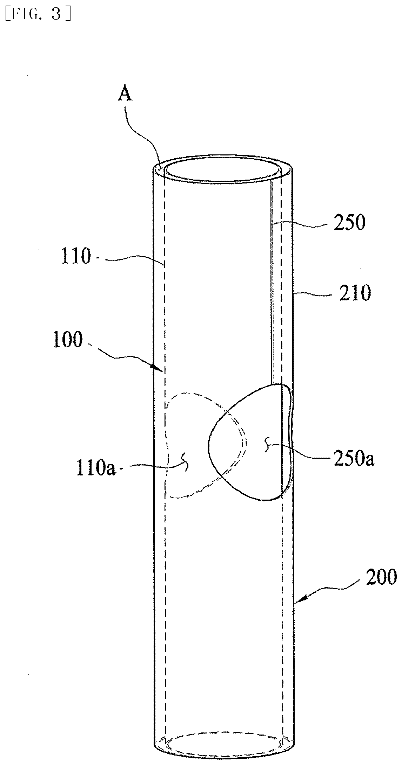

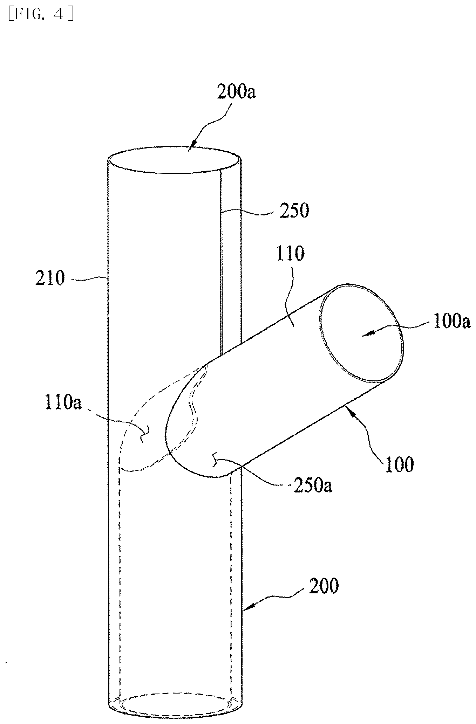

Referring to Figs. 3 and 4, the first induction conduit (100) may be inserted into the second induction conduit (200) to be formed integrally with the second induction conduit (200), or the front edge (100a) may be exposed from the second induction conduit (200) through a portion of the second induction conduit (200), i.e., the cut part (250), so that the first induction conduit (100) forms a Y-shaped duct together with the second induction conduit (200).

As explained above, when entering the bile duct (40), the first induction conduit (100) may enter the bile duct (40) together with the second induction conduit (200) in a state where the first induction conduit (100) is inserted into the second induction conduit (200) for easy entry.

When they arrive at the first branch bile duct (50) and the second branch bile duct (60) that are installation locations, the front edge (100a) of the first induction conduit (100) may be exposed from the second induction conduit (200) to the outside as in Fig. 4, so that the first induction conduit (100) and the second induction conduit (200) can be respectively installed in the first branch bile duct (50) and the second branch bile duct (60).

Meanwhile, the cut part (250) may be formed extending from a front edge (200a) toward a rear edge (200b) of the second induction conduit in the longitudinal direction, and a fixing hole (250a) penetrating the circumference part (210) may be formed on the cut part (250).

The cut part (250) of the second induction conduit (200) may be formed from the front edge (200a) to the rear edge (200b) of the circumference part (210) in the longitudinal direction or from the front edge (200a) of the circumference part (210) to the middle thereof.

The length of the cut part (250) is not limited, but the fixing hole (250a) may be formed in a section between the front edge (200a) and the rear edge (200b) of the circumference part (210).

In the present invention, for purposes of explanation, as an example, the cut part (250) is formed from the front edge (200a) of the second induction conduit (200) to the location where the fixing hole (250a) is formed.

The drainage catheter according to an example of the present invention may be formed, for example, of a plastic material and may be formed of a material with elasticity to be capable of elastic deformation and recovery. Accordingly, the cut part (250) formed in the second induction conduit (200) may be opened for the first induction conduit (100) to be exposed to the outside or closed tight again to seal the inner space of the circumference part (210).

Accordingly, when a force is applied to the inside of the cut part (250) by the first induction conduit (100), the cut part (250) may be opened. The force applied to the cut part (250) by the first induction conduit (100) may be by a first guide wire (300) to be described later.

As above, the front edge (100a) of the first induction conduit (100) may be exposed to the outside from the second induction conduit (200) through the cut part (250) and the front edge (100a) may be bent in the process of the exposure.

When inserted into the fixing hole (250a) of the second induction conduit (200) in the process of being bent, the front edge (100a) of the first induction conduit (100) may be fixed by the fixing hole (250a).

In this case, the fixing hole (250a) may be formed with a size corresponding to that of the first induction conduit (100), so that the fixing hole surrounds the first induction conduit (100).

Accordingly, when inserted into the fixing hole (250a) of the second induction conduit (200), the front edge (100a) of the first induction conduit (100) may be fixed by the fixing hole (250a) and not be bent any longer.

When the first induction conduit (100) is inserted into the fixing hole (250a), the cut part (250) may be closed tight again to seal the inner space of the circumference part (210).

Meanwhile, the first induction conduit (100) may have a hollow shaped body part (110) in contact with the circumference part (210) of the second induction conduit (200), and an opening hole (110a) may be formed on a face of the body part (110) in opposition to the penetrating hole (250a) of the second induction conduit (200).

When the front edge (100a) of the first induction conduit (100) is exposed from the second induction conduit (200), the opening hole (110a) formed on the first induction conduit (100) may increase in size as the first induction conduit (100) is bent and may be formed in communication with the inner space of the circumference part (210) of the second induction conduit.

Accordingly, the section other than the front edge (100a) of the first induction conduit (100), i.e., the section remaining in the second induction conduit (200), may be in communication with the second induction conduit (200) through the opening hole (110a).

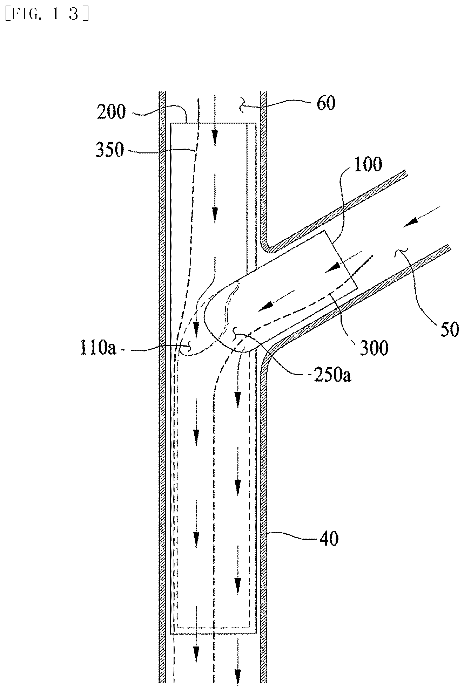

Referring to Fig. 13, when the first induction conduit (100) and the second induction conduit (200) are respectively installed in the first branch bile duct (50) and the second branch bile duct (60), bile may be introduced, through the first induction conduit (100) and the second induction conduit (200), to the region where the first induction conduit (100) and the second induction conduit (200) are coupled, and then be discharged.

That is, the opening hole (110a) allows the bile introduced through the second induction conduit (200) to be introduced into the first induction conduit (100) so as to be discharged out of the first induction conduit (100) together with the bile introduced through the first induction conduit (100).

Meanwhile, the drainage catheter according to an example of the present invention may include various modified examples of the first induction conduit (100) and the second induction conduit (200) to allow easy separation of the first induction conduit (100) from the second induction conduit (200).

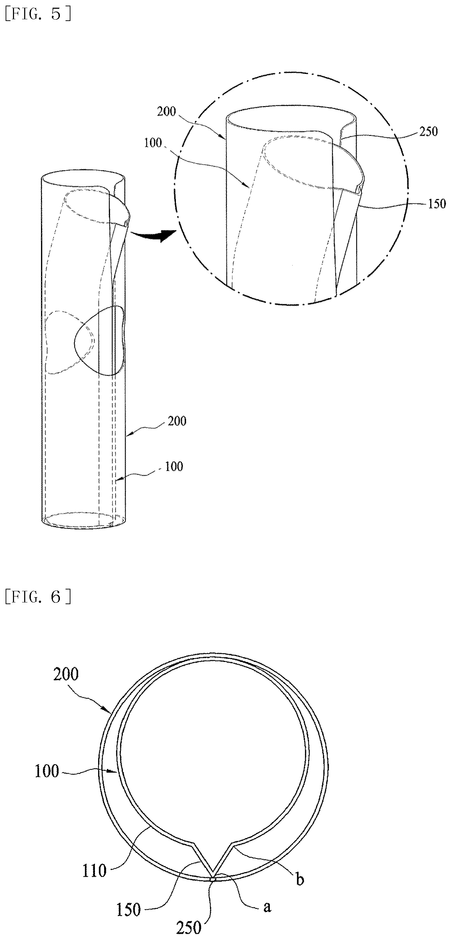

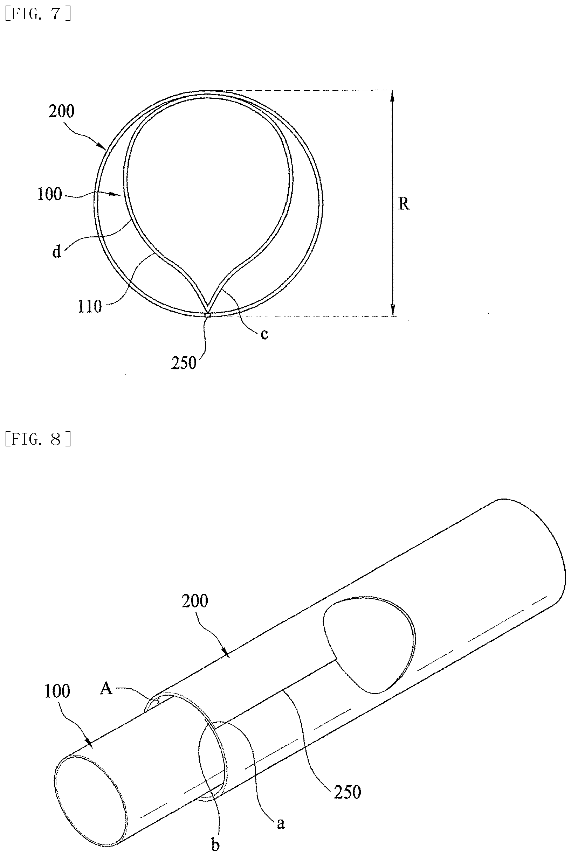

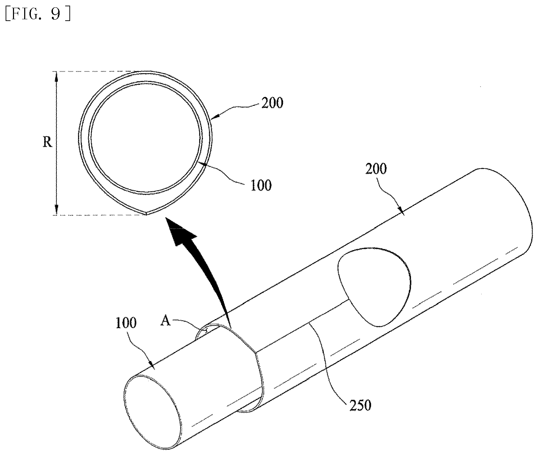

A modified example of the first induction conduit (100) is described with reference to Figs. 5 to 7, and a modified example of the second induction conduit (200) is described with reference to Figs. 8 and 9.

First, for a modified example of the first induction conduit (100), a protrusion part (150) may be formed in a region of the body part (110) of the first induction conduit (100), the region corresponding to the cut part (250) of the second induction conduit (200).

The protrusion part (150) may be formed in the longitudinal direction of the first induction conduit (100) and be formed to have a diameter that is smaller than that of the body part (110).

Accordingly, when the external force exposing the first induction conduit (100) from the second induction conduit (200) is the same, the force applied to the cut part (250) by the protrusion part (150) may become greater than the force applied to the cut part (250) by the body part (110).

Accordingly, due to the formation of the protrusion part (150), the cut part (250) may be more easily opened so that the first induction conduit (100) may be easily exposed to the outside from the second induction conduit (200).

As above, the pressure applied to the cut part (250) may vary depending on the size of the diameter of the protrusion part (150). Referring to Fig. 6, the protrusion part (150) may be formed such that the width of an edge (a) contacting the cut part (250) is narrower than that of the other edge (b) connected to the body part (110).

As above, as the width of the edge (a) of the protrusion part (150) contacting the cut part (250) is narrower, the pressure applied to the cut part (250) may increase. Accordingly, the cut part (250) may be easily opened.

However, the cut part (250), which is easily opened when an external force is applied by the first induction conduit (100), may be formed to be closed again to seal the inner space of the circumference part (210) when the external force is removed because the first induction conduit (100) is inserted into the fixing hole (250a).

Meanwhile, no protrusion part (150) may be formed in the first induction conduit (100), and the first induction conduit (100) itself may be deformed to have the cut part (250) easily opened.

Referring to Fig. 7, the body part (110) of the first induction conduit (100) may be formed such that a side (c) contacting the cut part (250) is protruded outwardly in comparison to the other side (d) that is opposite in the radial direction (R).

Accordingly, the side (c) of the body part (110) contacting the cut part (250) may be formed to have a diameter that is smaller than that of the other side (d) to increase the pressure applied to the cut part (250).

As above, in the catheter according to an example of the present invention, the first induction conduit (100) may be modified in a variety of ways to have the cut part (250) opened by a smaller force to easily expose the first induction conduit (100).

Further, two modified examples of the cut part (250) are described with reference to Figs. 8 and 9.

Referring to Fig. 8, the circumference part (210) of the second induction conduit (200) may be formed such that a face (a) constituting the cut part (250) overlaps onto an upper portion of the other face.

Or, as in Fig. 9, the circumference part (210) of the second induction conduit (200) may be formed such that a side on which the cut part (250) is formed is protruded outwardly in comparison to the other side that is opposite in the radial direction (R).

The modified examples of the cut part (250) are aimed to enhancing a function of sealing the inner space of the circumference part (210) without disturbing the exposure of the first induction conduit (100).

As above, in the drainage catheter according to an example of the present invention, the cut part (250) may be formed in the second induction conduit (200) so that the first induction conduit (100) is easily exposed while enhancing the sealing of the inner space of the second induction conduit (200).

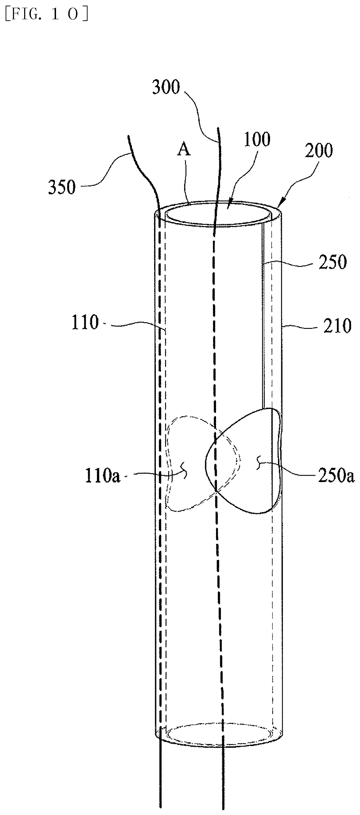

Meanwhile, referring to Fig. 10, a first guide wire (300) and a second guide wire (350) may be further included.

The first guide wire (300) may be inserted into the body part (110) of the first induction conduit (100) and exposed from the body part (110) to guide the first induction conduit (100) to an installation location.

The second guide wire (350) may be inserted into a space (A) between the circumference part (210) of the second induction conduit (200) and the body part (110) and exposed from the circumference part (210) to guide the second induction conduit (200) to an installation location.

The first guide wire (300) and the second guide wire (350) search for the first branch bile duct (50) and the second branch bile duct (60) while the drainage catheter according to an example of the present application is being installed at the installation locations, such that the first induction conduit (100) and the second induction conduit (200) can be accurately installed at the installation locations.

Specifically, the installation processes of the first induction conduit (100) and the second induction conduit (200) are described.

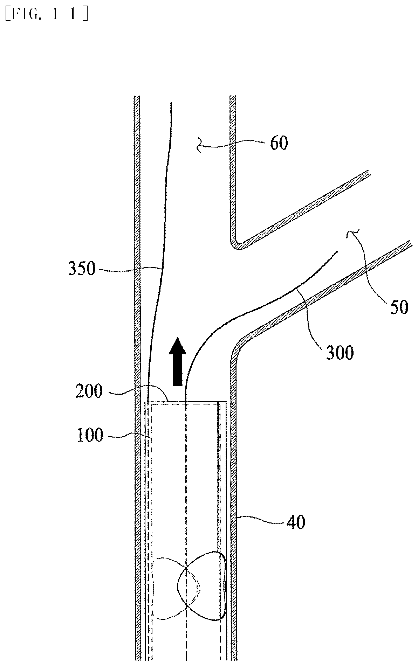

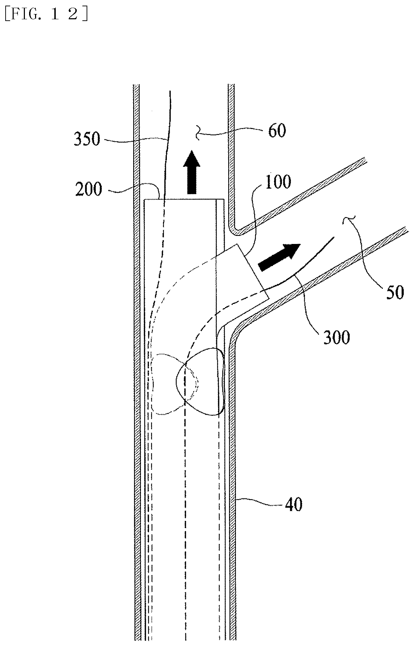

Referring to Figs. 11 to 13, the locations where the drainage catheter according to an example of the present invention is to be installed are the second branch bile duct (60) connected to the bile duct (40) and the first branch bile duct (50) diverged from the bile duct (40).

First, the first induction conduit (100) and the second induction conduit (200) may enter the bile duct (40) in a coupled state.

In this case, the first guide wire (300) and the second guide wire (350) are exposed to the outside from the front edges of the first induction conduit (100) and the second induction conduit (200) and thus may enter the bile duct (40) before the first induction conduit (100) and the second induction conduit (200).

When the first guide wire (300) and the second guide wire (350) reach the locations of the first branch bile duct (50) and the second branch bile duct (60) before the first induction conduit (100) and the second induction conduit (200), the first guide wire (300) may be adjusted to be directed to the first branch bile duct (50) and the second guide wire (350) may be adjusted to be directed to the second branch bile duct (60).

Accordingly, the first induction conduit (100) is moved following the first guide wire (300) to the first branch bile duct (50) and the second induction conduit (200) is moved following the second guide wire (350) to the second branch bile duct (60).

That is, by following the first guide wire (300) to the first branch bile duct (50), the first induction conduit (100) may be separated from the second induction conduit (200) through the cut part (250) of the second induction conduit (200).

The first induction conduit (100) and the second induction conduit (200) continue to move forward, and when the first induction conduit (100) is coupled to the fixing hole (250a) of the second induction conduit (200), no further movement is made and, accordingly, the installation of the first induction conduit (100) and the second induction conduit (200) may be completed.

When the installation of the first induction conduit (100) and second induction conduit (200) is completed, the front edge (100a) of the first induction conduit (100) may be located in the first branch bile duct (50), the front edge (200a) of the second induction conduit (200) may be located in the second branch bile duct (60), and the other portion of the first induction conduit (100) and the other portion of the second induction conduit (200) may be located in the bile duct (40) in a state of being coupled and in mutual communication.

After introduced into the first induction conduit (100), bile in the first branch bile duct (50) may pass through the inner space of the first induction conduit (100) to be discharged to the bile duct (40).

Further, after introduced into the second induction conduit (200), bile in the second branch bile duct (60) may be introduced to the inner space of the first induction conduit (100) through the opening hole (110a) of the first induction conduit (100) and then pass through the first induction conduit (100) to be discharged to the bile duct (40).

Accordingly, by way of the first induction conduit (100) and the second induction conduit (200), bile in the first branch bile duct (50) and second branch bile duct (60) may be smoothly discharged to the bile duct (40).

Examples of the present invention are described as above, and the fact that the present invention can be embodied in other specific forms in addition to the above-described examples without departing from the spirit or scope of the present invention will be obvious to those having ordinary skill in the pertinent field. Therefore, the above-described examples should be regarded as illustrative rather than restrictive, and thus, the present invention is not limited to the above description and may be modified within the scope of the appended claims and their equivalents.

### [Description of reference numerals]

10: liver; 20: gallbladder
30: pancreas; 40: bile duct(s)
50: first branch bile duct; 60: second branch bile duct
100: first induction conduit; 100a: front edge
110: body part; 110a: opening hole
150: protrusion part; 200: second induction conduit
200a: front edge; 200b: rear edge (200b)
210: circumference part; 250: cut part
250a: fixing hole; 300: first guide wire
350: second guide wire; A: separated space
a: face b: the other face
c: side d: the other side
R: radial direction

## Claims

1. A drainage catheter comprising:
a first induction conduit; and
a second induction conduit having a circumference part surrounding the first induction conduit, in which a cut part is formed so that a front edge of the first induction conduit is exposed to the outside.

2. The drainage catheter according to claim 1,
wherein the cut part is formed extending from a front edge toward a rear edge of the second induction conduit in the longitudinal direction, and
a fixing hole penetrating the circumference part is formed on the cut part.

3. The drainage catheter according to claim 2,
wherein the fixing hole is formed with a size corresponding to that of the first induction conduit, so that the fixing hole surrounds the first induction conduit.

4. The drainage catheter according to claim 2,
wherein the first induction conduit has a hollow shaped body part in contact with the circumference part of the second induction conduit, and
an opening hole is formed on a face of the body part in opposition to the penetrating hole of the second induction conduit.

5. The drainage catheter according to claim 4,
wherein a protrusion part is formed in a region of the body part of the first induction conduit, the region corresponding to the cut part of the second induction conduit.

6. The drainage catheter according to claim 5,
wherein the protrusion part is formed such that the width of an edge contacting the cut part is narrower than that of the other edge connected to the body part.

7. The drainage catheter according to claim 4,
wherein the body part of the first induction conduit is formed such that a side contacting the cut part is protruded outwardly in comparison to the other side that is opposite in the radial direction.

8. The drainage catheter according to claim 1,
wherein the circumference part of the second induction conduit is formed such that a face constituting the cut part overlaps onto an upper portion of the other face.

9. The drainage catheter according to claim 1,
wherein the circumference part of the second induction conduit is formed such that a side on which the cut part is formed is protruded outwardly in comparison to the other side that is opposite in the radial direction.

10. The drainage catheter according to claim 1,
wherein the first induction conduit has a hollow shaped body part in contact with the circumference part of the second induction conduit, and
wherein the drainage catheter further comprises:
a first guide wire which is inserted into the body part and is exposed from the body part to guide the first induction conduit to an installation location; and
a second guide wire which is inserted into a separated space between the circumference part and the body part and is exposed from the circumference part to guide the second induction conduit to an installation location.
